# EUROPEAN PATENT APPLICATION

(11) **EP 3 001 916 A1**
(43) Date of publication of application: **06.04.2016**
(21) Application number: 15182906.6
(22) Date of filing: 28.08.2015
(51) Int. Cl.: A23L 33/115, A23L 33/17, A23L 17/60, A23L 29/10

(54) **FOOD OR DRINK COMPRISING AN ALGAE EXTRACT OIL**

(30) Priority: 02.10.2014 JP 2014203796
(71) Applicant: Nikken Kagaku Co., Ltd., Gifu 501-6255 (JP)
(72) Inventor: MORI, Nobuo, Hashima-shi, Gifu 5016255 (JP); KATSUYAMA, Hidenori, Hashima-shi, Gifu 5016255 (JP); SUMI, Ryo, Hashima-shi, Gifu 5016255 (JP)
(74) Representative: Bailey, Sam Rogerson

(57) **Abstract**

The present invention provides a food or drink that emits no offensive odor typified by a fish odor in any form of the food or drink, such as liquid, gel, semi-solid, or solid form, and also suppresses emission of an offensive odor due to time-dependent changes. Further, a food or drink is provided that has, particularly, a superior action of inhibiting platelet aggregation to that of conventional fish-derived EPA and DHA-containing food. The food or drink comprise an algae extract oil, an animal or plant protein, and an emulsifier.

## Description

### BACKGROUND OF THE INVENTION

### Field of the Invention

The present invention relates to a food or drink that contains an algae extract oil.

### Description of the Related Art

There have heretofore existed many foods or drinks that contain unsaturated fatty acids such as eicosapentaenoic acid (EPA) and docosahexaenoic acid (DHA).

It is known that EPA and DHA have physiological activation effects such as actions of inhibiting platelet aggregation, of reducing blood triglyceride, and of reducing blood VLDL and LDL cholesterols, and have prophylactic and therapeutic effects on arteriosclerotic diseases, and the like.

An example of the foods or drinks that contain EPA and DHA is a soy milk that contains a high concentration of EPA and/or DHA derived from a fish oil. It is disclosed that the fish oil used in the soy milk is a fish oil which contains 10% or more of EPA, preferably 25% or more of EPA and DHA together, or 28% or more of EPA (Japanese Patent No. 4738714).

Another example of such foods or drinks is an acid milk that contains EPA and/or DHA derived from a fish oil and has excellent oxidation and emulsion stability. It is disclosed that the acid milk, to which an acid has been added to decrease its acidity to pH 4.5 or less, has oxidation and emulsion stability at 5°C for 15 days or more (Japanese Patent No. 4202130).

However, it is known that fish oils, which contain EPA and DHA, have a distinct offensive odor, are extremely oxidation sensitive and subjected to degradation in the flavor to thereby exhibit unpleasant odors and tastes.

Nevertheless, it is disclosed that the soy milk and acid milk exhibit no fish odor derived from the fish oil used as the raw material, have a good taste, smell, and mouthfeel together, and also have good preservability of EPA and DHA.

### SUMMARY OF THE INVENTION

The soy milk and acid milk described in Japanese Patent Nos. 4738714 and 4202130 described above both exhibit no fish odor derived from the fish oil used as the raw material and have a good taste, smell, and mouthfeel together. However, it is clear that an increase in the content of the fish oil causes an fish odor to be exhibited as long as the fish oil is used as the raw material. Therefore, even if a high concentration of EPA and/or DHA is contained, the high concentration has a limit.

Additionally, the cases in Japanese Patent Nos. 4738714 and 4202130 described above are limited to soy milk and acid milk. It is not ensured that even another food or drink that contains fish oil exhibits no fish odor derived from the fish oil.

The present invention is thus intended to solve the conventional problem described above, and it is an object of the present invention to provide a food or drink that emits no offensive odor typified by a fish odor in any form of the food or drink, such as liquid, gel, semi-solid, or solid form, and also suppresses emission of an offensive odor due to time-dependent changes.

It is another object of the present invention to provide a food or drink that has, particularly, a superior action of inhibiting platelet aggregation to that of a conventional fish-derived EPA and DHA-containing food.

The food or drink of the present invention contains an algae extract oil, an animal or plant protein, and an emulsifier.

The algae extract oil in the food or drink of the present invention is preferably one extracted from Labyrinthulea of microalgae.

The animal or plant protein in the food or drink of the present invention is preferably at least one selected from milk protein, soybean protein, and collagen peptide.

The algae extract oil in the food or drink of the present invention contains 30 to 50 wt% of DHA and 1 to 20 wt% of DPA.

The algae extract oil in the food or drink of the present invention further contains 0.1 to 1 wt% of EPA.

The food or drink of the present invention, in any form such as liquid, gel, semi-solid, or solid form, emits no offensive odor typified by a fish odor and also suppress emission of an offensive odor due to time-dependent changes.

Furthermore, it has been found that the food or drink of the present invention has a superior action of inhibiting platelet aggregation to that of a conventional fish-derived EPA and DHA-containing food.

### BRIEF DESCRIPTION OF THE DRAWINGS

FIG. 1 is a graph showing the sensory test of odors from an algae extract oil used in the food or drink of the present invention and from a commercially available fish oil;
FIG. 2 is a graph showing the platelet aggregation inhibition ratio of the algae extract oil used in the food or drink of the present invention and of the commercially available fish oil (blood coagulation factor: adenosine diphosphate);
FIG. 3 is a graph showing the platelet aggregation inhibition ratio of the algae extract oil used in the food or drink of the present invention and of the commercially available fish oil (blood coagulation factor: arachidonate); and
FIG. 4 is a graph showing the platelet aggregation inhibition ratio of the algae extract oil used in the food or drink of the present invention and of the commercially available fish oil (blood coagulation factor: PAF).

### DETAILED DESCRIPTION OF THE PREFERRED EMBODIMENTS

Embodiments to implement the food or drink of the present invention will be described in detail hereinbelow.

The food or drink of the present invention contains an algae extract oil, an animal or plant protein, and an emulsifier.

The algae extract oil is contained in an amount of 1 to 30 wt% in the food or drink of the present invention. The algae extract oil contains 30 to 50 wt% of docosahexaenoic acid (DHA) and 1 to 20 wt% of docosapentaenoic acid (DPA). The algae extract oil preferably contains 35 to 45 wt% of DHA and 10 to 15 wt% of DPA. The algae extract oil further contains 0.1 to 1 wt% of eicosapentaenoic acid (EPA) in addition to DHA and DPA.

Preferred algae extract oils are those extracted from Aurantiochytrium sp., Schizochytrium sp. and the like of Labyrinthulea of microalgae. Extraction of an oil from these microalgae can provide an extract oil having a high content of docosahexaenoic acid (DHA) and docosapentaenoic acid (DPA). A specific example of such algae extract oil includes an algae extract oil (trade name: DHA rich oil) manufactured by Vedan Biotechnology Corporation in Taiwan.

The animal or plant protein is preferably contained in an amount of 2 to 50 wt% in the food or drink of the present invention. The animal or plant protein is preferably at least one selected from milk protein, soybean protein, and collagen peptide.

Examples of the milk protein that can be used in the food or drink of the present invention include, but are not particularly limited to, milk, skim milk, concentrated milk, concentrated skim milk, whole milk powder, skim milk powder, condensed milk, defatted condensed milk, soy milk, yogurt, cheese, butter, buttermilk, cream, cream powder, concentrated whey, and whey powder.

Examples of the soybean protein that can be used in the food or drink of the present invention may be any protein contained in soybeans, and include, but are not particularly limited to, soy milk, concentrated soybean protein, isolated soybean protein, and soybean peptide. Examples of such soybean protein that can be used include those commercially available as supplement food and materials for food and drink.

Examples of the collagen peptide that can be used in the food or drink of the present invention include those provided by hydrolysis of collagen or gelatin with an enzyme or acid, without particular limitation, and preferably having an average molecular weight of the order of 100 to 300,000. Examples of such collagen peptide that can be used include those commercially available as materials for food and drink both in powder and liquid forms.

The emulsifier is preferably contained in an amount of 80 to 300 wt% relative to the algae extract oil. The emulsifier is preferably at least one selected from glycerin fatty acid esters, sucrose fatty acid esters, soy lecithin, yolk lecithin, and the like.

The food or drink of the present invention may contain one or more of water, fragrance, a taste-masking agent, seasoning, an organic acid, a pH adjusting agent, and the like in addition to the algae extract oil, the animal or plant protein, and the emulsifier.

Examples of the fragrance include yogurt fragrance, lemon fragrance, pineapple fragrance, grapefruit fragrance, lemon lime fragrance, orange fragrance, and apple fragrance.

Examples of the taste-masking agent include green tea, black tea, oolong tea, coffee, almond oil, peppermint oil, spearmint oil, cinnamon oil, and menthol.

Examples of the seasoning include salt, sugar, sweet cooking rice wine, Japanese sake, and wine.

Examples of the organic acid include lactic acid, malic acid, citric acid, tartaric acid, and ascorbic acid.

Examples of the pH adjusting agent include the organic acids and its salts, phosphoric acid and its salts, and carbonic acid and its salts.

Subsequently, a method for producing the food or drink of the present invention will be outlined.

In a first step, an algae extract oil and an emulsifier are mixed. The mixture ratio between the algae extract oil and the emulsifier is 1:0.1 to 1:50.

In a second step, to the mixture of the algae extract oil and the emulsifier obtained in the first step, water or hot water is added and stirred until emulsification takes place. The mixture ratio may be any value, and the temperature of the water or hot water is 5 to 100°C. Additionally, the stirring may be continued for any length of time, which is preferably between 5 minutes and 24 hours.

In a third step, to the emulsion obtained in the second step, milk protein and/or soybean protein and/or collagen peptide, which is an animal or plant protein, is/are added, and additionally mixed and stirred. The mixture ratio between the emulsion and the animal or plant protein is 1:0.1 to 1:500. Any stirring temperature, stirring time, and stirring method may be used.

To the emulsion mixture obtained via such steps, vitamin C, citric acid, tartaric acid, and the like are added to adjust the pH to 1.5 to 5 and make the mixture an acidic liquid.

Then, the acidic liquid may be added to milk or soy milk to provide a beverage. To the acidic liquid, as it is or diluted, fruit juice and the like may be added to provide a beverage.

Furthermore, the acidic liquid, as it is or diluted, may be blended to produce food such as retort food, baked confectionery such as cookies and rice crackers.

Alternatively, to the acidic liquid, as it is or diluted, a thickener and a polysaccharide may be added to produce gelled food or jelly-like semi-solidified or solidified food.

Subsequently, the food or drink of the present invention will be described in detail referring to examples, but the food or drink of the present invention are not intended to be limited by these examples.

### (Evaluation of suppression of offensive odor emission due to time-dependent changes)

In order to evaluate suppression of offensive odor emission due to time-dependent changes using an algae extract oil used in the food or drink of the present invention and a commercially available fish oil, monitor test was carried out as described below (for a period of about one month) by 10 males and females.

The algae extract oil used was an algae extract oil (trade name: DHA rich oil) manufactured by Vedan Biotechnology Corporation (Taiwan). The fish oil used was a purified fish oil commonly used as food.

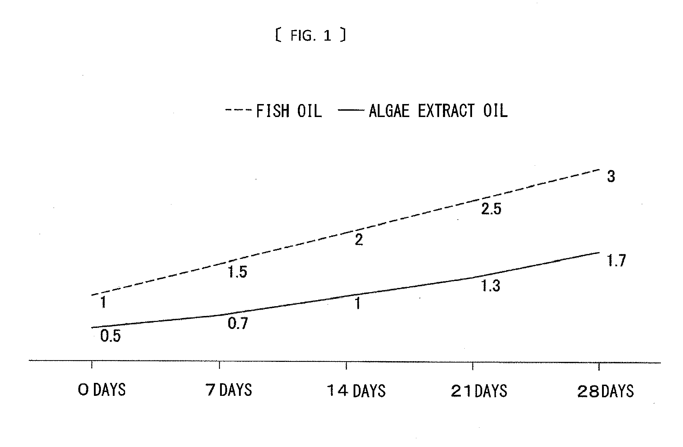

Ten males and females were asked to smell the odor of the algae extract oil and the fish oil once a week for four weeks. In accordance with the following evaluation criteria, the degree of the offensive odor emission was scored and the average was determined. The results are shown in FIG. 1.

The evaluation criteria included three grades: "0": No odor is noticed; "1": An odor is barely noticed; "2": An odor is slightly noticed; and "3": An odor is clearly noticed.

As the results of the monitor test, after two weeks, the degree of the offensive odor emission from the algae extract oil was scored "1", and the degree of the offensive odor emission from the fish oil was scored "2". Numerically, the degree of the offensive odor emission from the algae extract oil was 50% of that from the fish oil. Furthermore, after four weeks, the degree of the offensive odor emission from the algae extract oil was scored "1.7", and the degree of the offensive odor emission from the fish oil was scored "3". Numerically, the degree of the offensive odor emission from the algae extract oil was about 57% of that from the fish oil.

### (Confirmation of the action of inhibiting platelet aggregation)

One algae extract oil used in the food or drink of the present invention and two commercially available fish oils were used to prepare drinkable preparations, which were used to confirm the action of inhibiting platelet aggregation.

The algae extract oil used to be contained in a drinkable preparation was DHA rich oil (DHA: 38 wt% and EPA: 0.4 wt%) manufactured by Vedan Biotechnology Corporation (Taiwan), and the fish oils to be contained in a drinkable preparation were a purified fish oil, DHA 27 oil (DHA: 27 wt% and EPA: 8 wt%) commonly used as food and a purified fish oil, DHA 46 oil (DHA: 46 wt% and EPA: 5 wt%) commonly used.

In sample preparation, human blood was centrifuged to collect platelet-rich plasma (PRP), which was centrifuged to collect platelet-poor plasma (PPP).

In addition, blood coagulation factors (adenosine diphosphate, an arachidonate salt, and platelet-activating factor (PAF)) were each added to the PRP to aggregate platelets.

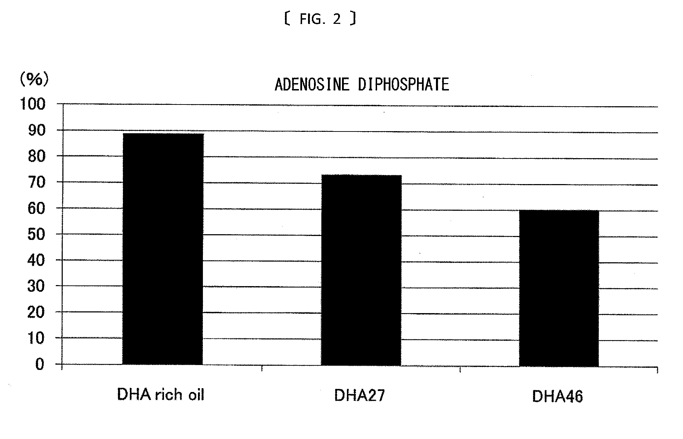

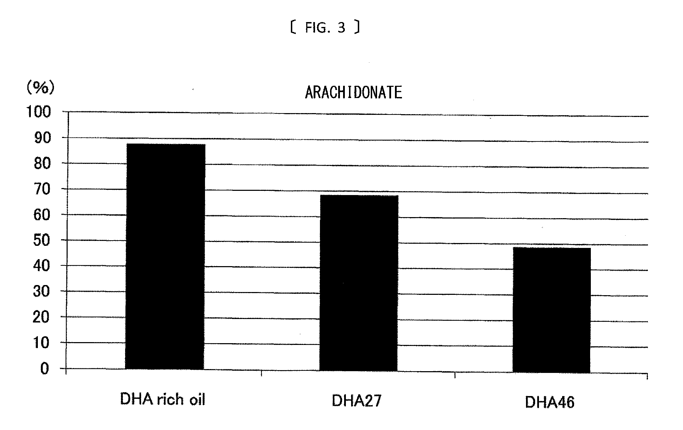

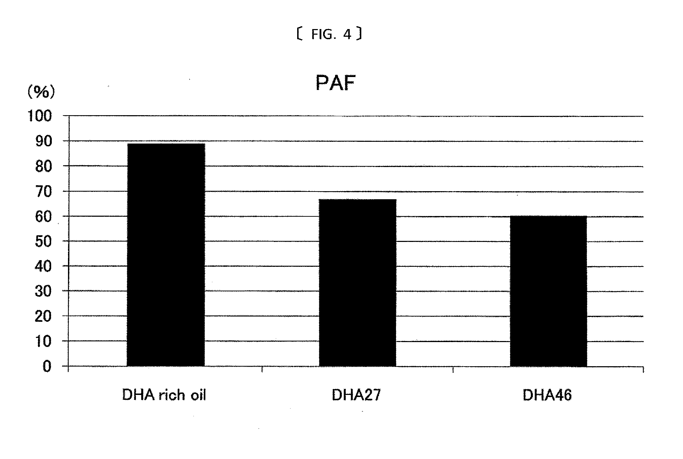

Each DHA oil was added to each sample described above, and the light transmittance of PRP was set at 0% and the light transmittance of PPP was set at 100% before addition of an aggregating agent. The maximum transmittance of PRP after addition of the aggregating agent was taken as the maximum percentage of aggregation (%), i.e., the percentage of platelet aggregation (%). The averaged results of testing in triplicate are shown in FIGS. 2 to 4.

The results of testing for confirmation of the action of inhibiting platelet aggregation are as follows.

The percentage of platelet aggregation of the algae extract oil used in the food or drink of the present invention was 89% for adenosine diphosphate, 88% for the arachidonate, and 89% for PAF.

The percentage of platelet aggregation of the two commercially available fish oils were 73% and 60% for adenosine diphosphate, 69% and 49% for the arachidonate, 67% and 60% for PAF.

Examples of the food or drink of the present invention as a drinkable preparation or jelly-like food will be described hereinbelow.

### [Example 1]

### (Drinkable preparation ingredients)

| | |
|---|---|
| Algae extract oil (Vedan Biotechnology Corporation: DHA rich oil) | 3,000 mg |
| Glycerin fatty acid ester (emulsifier) | 3,600 mg |
| Sucralose (50-times liquid) | 1,000 mg |
| Citric acid | 10 mg |
| Collagen peptide | 5,000 mg |
| Yogurt fragrance | 400 mg |
| Vanilla flavor | 100 mg |
| Purified water | 86,790 mg |

A drinkable preparation containing the ingredients described above was prepared in accordance with the production method aforementioned. The drinkable preparation was placed in a bottle, which was capped and stored in a refrigerator (inner temperature: 10°C) for one month, after which the bottle was opened and the odor was smelled. No odor other than the yogurt fragrance and vanilla flavor was felt at all.

The drinkable preparation contains DHA and EPA, which have an excellent action of inhibiting platelet aggregation, derived from the algae extract oil. Thus, drinking the preparation for a long period holds promise of prophylactic and therapeutic effects on arteriosclerotic diseases.

### [Example 2]

### (Jelly-like food ingredients)

| | |
|---|---|
| Algae extract oil (Vedan Biotechnology Corporation: DHA rich oil) | 100 mg |
| Glycerin fatty acid ester (emulsifier) | 200 mg |
| Sucralose (50-times liquid) | 150 mg |
| Citric acid | 90 mg |
| Collagen peptide | 400 mg |
| Yogurt fragrance | 30 mg |
| Pink grapefruit fragrance | 40 mg |
| Trehalose | 2,000 mg |
| Thickening polysaccharide (gelling agent) | 150 mg |
| Maltodextrin | 1,700 mg |
| Glycerin fatty acid ester (antifoaming agent) | 2 mg |
| Collagen masking fragrance | 50 mg |
| Sodium citrate | 60 mg |
| Purified water | 5,028 mg |

Jelly-like food containing the ingredients described above was prepared in accordance with the production method aforementioned. The jelly-like food was placed in a synthetic resin container, which was capped and stored in a refrigerator (inner temperature: 10°C) for one month, after which the container was opened and the odor was smelled. No odor other than the yogurt fragrance and pink grapefruit was felt at all.

The jelly-like food contains DHA and EPA derived from the algae extract oil which has an excellent action of inhibiting platelet aggregation. Thus, taking the food for a long period holds promise of prophylactic or therapeutic effect on arteriosclerotic disease.

## Claims

1. A food or drink comprising an algae extract oil, an animal or plant protein, and an emulsifier.

2. The food or drink according to claim 1, wherein the algae extract oil is one extracted from Labyrinthulea of microalgae.

3. The food or drink according to claim 1 or 2, wherein the animal or plant protein is at least one selected from milk protein, soybean protein, and collagen peptide.

4. The food or drink according to any of claims 1 to 3, wherein the algae extract oil comprises 30 to 50 wt% of DHA and 1 to 20 wt% of DPA.

5. The food or drink according to claim 4, wherein the algae extract oil further comprises 0.1 to 1 wt% of EPA.
